# EUROPÄISCHE PATENTSCHRIFT

(11) **EP 1 363 956 B1**
(45) Veröffentlichungstag und Bekanntmachung des Hinweises auf die Patenterteilung: **11.03.2015**
(21) Anmeldenummer: 01991763.2
(22) Anmeldetag: 28.11.2001
(51) Int. Cl.: C08F 265/00, C08F 265/10, C08F 290/06, C08F 291/00, C08L 51/00, C08F 220/58, A61K 8/81, A61Q 19/00, C08F 265/04

(54) **KAMMFÖRMIGE COPOLYMERE AUF BASIS VON ACRYLOYLDIMETHYLTAURINSÄURE (2-ACRYLAMIDO-2-METHYL-1-PROPANSULFONSÄURE)**
COMB-SHAPED COPOLYMERS BASED ON ACRYLOYLDIMETHYL TAURINE ACID
COPOLYMERES EN PEIGNE A BASE D'ACIDE D'ACRYLOYLDIMETHYLTAURINE

(30) Priorität: 01.12.2000 DE 10059828
(43) Veröffentlichungstag der Anmeldung: 26.11.2003
(73) Patentinhaber: Clariant Produkte (Deutschland) GmbH, 65929 Frankfurt am Main (DE)
(72) Erfinder: MORSCHHÄUSER, Roman, 55122 Mainz (DE); GLAUDER, Jan, 65812 Bad Soden (DE); LÖFFLER, Matthias, 65527 Niedernhausen (DE); KAYSER, Christoph, 55127 Mainz (DE); TARDI, Aranka, 63543 Neuberg (DE)
(86) Internationale Anmeldenummer: PCT/EP2001/013854
(87) Internationale Veröffentlichungsnummer: WO 2002/044224

(56) Entgegenhaltungen:
- EP-A- 0 356 241
- EP-A- 0 522 756
- EP-A- 1 069 142
- EP-A- 1 083 184
- WO-A-99/04750
- DE-A- 19 907 587
- DE-A- 19 951 877
- US-A- 4 859 458
- US-A- 5 379 841
- US-A- 5 837 789

## Beschreibung

Die vorliegende Erfindung betrifft Copolymere auf Basis von Acryloyldimethyltaurinsäure bzw. Acryloyldimethyltauraten.

In den letzten Jahren erlangten wasserlösliche Polymere eine immer größer werdende Bedeutung in Industrie und Wissenschaft. Polyelektrolyte nehmen dabei mengenmäßig einen sehr großen Teil der jährlichen Gesamtproduktion ein. Sie finden z.B. Anwendung in der Papierverarbeitung, der Waschmittelindustrie, der Textilverarbeitung, der Erdölgewinnung oder als wichtige Kosmetikrohstoffe.
Im kosmetischen Bereich kommt Polyelektrolyten eine tragende Rolle zu. Neben wasserlöslichen, oberflächenaktiven Stoffen gibt es in diesem Bereich einen hohen Bedarf an wasser- und ölverdickenden Systemen. Derartige Verdicker, insbesondere die auf Basis der Polyacrylsäure hergestellten "Superabsorber", sind seit ihrer Entwicklung in den 70iger Jahren aus dem Hygienebereich nicht mehr wegzudenken. In ihren vernetzten Varianten werden teil- oder vollneutralisierte Polyacrylsäuren und deren wasserlösliche Copolymere in vielen Kosmetikformulierungen als Konsistenzgeber eingesetzt. Die Vielfalt der möglichen Strukturen und die damit verbundenen vielfältigen Anwendungsmöglichkeiten drücken sich nicht zuletzt in einer Vielzahl von Patenten aus, die seit Mitte der 70iger Jahre weltweit angemeldet wurden.
In den 90iger Jahren wurden neuartige Verdicker auf Basis von 2-Acrylamido-2-methyl-1-propansulfonsäure (AMPS) bzw. deren Salzen in den Markt eingeführt (EP 816 403 WO9904750, DE19907587, EP1069142, US5837789, und WO 98/00 094). Sowohl als Homo- als auch in Form der Copolymere (^{®}Aristoflex AVC, Clariant GmbH) sind derartige Verdicker den entsprechenden Polycarboxylaten (Carbopole) in vieler Hinsicht überlegen. Beispielsweise zeigen Verdickersysteme auf Basis von AMPS hervorragende Eigenschaften in pH-Bereichen unterhalb von pH 6, also in einem pH-Bereich, in dem mit herkömmlichen Polycarboxylat-Verdickern nicht mehr gearbeitet werden kann. Zudem führt die den Acryloyldimethyltaurinsäure-Verdickern eigene Mikrogelstruktur zu einem besonders angenehmen Hautgefühl. Die leichte Verarbeitbarkeit und das günstige toxikologische Profil des Hauptmonomeren verleihen diesen Verdickern ein hohes Anwendungspotential.

Im Laufe der letzten Jahre drängten Vertreter eines neuen Verdickerkonzeptes auf den Markt. Hierbei wurden in einem Polymer zwei unterschiedliche Eigenschaften kombiniert und damit neue Anwendungsfelder erschlossen. Verdickende Emulgatoren oder Dispergatoren sind nur zwei Beispiele dieser neuen Substanzklasse. Als Markennamen können die Pemulene^{®} TR-1 und TR-2 von BF-Goodrich oder die Aculyn^{®}-Typen von Rohm und Haas genannt werden. Alle bisherigen Varianten basieren auf hydrophob modifizierten Varianten der herkömmlichen Polyacrylate.

Überraschend wurde gefunden, daß eine neue Klasse von Copolymeren auf Basis der Acryloyldimethyltaurinsäure (AMPS) dem Anwender die Möglichkeit gibt, Eigenschaften von unpolaren Molekülteilen, wie Alkylketten oder silikonhaltigen Strukturen, mit hydrophilen Struktureinheiten, wie Polyglykolen oder Polyelektrolyten, zu kombinieren, was ihm wiederum völlig neue Möglichkeiten in der Formulierungstechnik gibt. Der Einsatz der erfindungsgemäßen, multifunktionalen Polymeren ermöglicht beispielsweise Herstellern von Kosmetika eine deutliche Vereinfachung der Rezepturen.

Gegenstand der Erfindung sind wasserlösliche oder wasserquellbare Copolymere, erhältlich durch radikalische Copolymerisation von
A) Acryloyldimethyltaurinsäure und/oder Acryloyldimethyltauraten, wobei der Neutralisationsgrad oberhalb von 80 % ist,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen, und bei denen es sich um [2-(Methacryloyloxy)ethyl]trimethylammoniumchlorid, [2-(Acryloyloxy)ethyl]trimethylammoniumchlorid, [2-Methacrylamido-ethyl]trimethylammoniumchlorid, [2-(Acrylamido)ethyl]trimethyl-ammoniumchlorid, N-Methyl-2-vinylpyridiniumchlorid und/oder N-Methyl-4-vinylpyridiniumchlorid handelt,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation in Gegenwart mindestens eines polymeren Additivs mit zahlenmittleren Molekulargewichten von 200 g/mol bis 10⁹ g/mol, bei denen es sich um Homo- oder Copolymere aus N-Vinylformamid, N-Vinylacetamid, N-Vinylpyrrolidon, Ethylenoxid, Propylenoxid, Acryloyldimethyltaurinsäure, N-Vinylcaprolacton, N-Vinylmethylacetamid, Acrylamid, Acrylsäure, Methacrylsäure, N-Vinylmorpholid, Hydroxymethylmethacrylat, Diallyldimethylammonium-chlorid (DADMAC) und/oder [2-(Methacryloyloxy)ethyl]trimethylammoniumchlorid; Polyalkylenglykole und/oder Alkylpolyglykole handelt, erfolgt,
H) mit der Maßgabe, dass die Komponente A) mit mindestens zwei Komponenten ausgewählt aus mindestens zwei der Gruppen C) bis F) copolymerisiert wird,
dadurch gekennzeichnet, dass der Gehalt an Acryloyldimethyltaurinsäure bzw. Acryloyldimethyltauraten, bezogen auf die Gesamtmasse der Copolymere, 50 bis 99,5 Gew.-% beträgt.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure handeln. Bevorzugt werden die Li⁺-, Na⁺-, K⁺-, Mg⁺⁺-, Ca⁺⁺-, Al⁺⁺⁺- und/oder NH4⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure ist ein Neutralisationsgrad von oberhalb 80%.
Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.
Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 22.
Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.
Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C_{1 -} C₂₂)-Alkylreste oder (C₂ - C₁₀)-Hydroxyalkylreste handeln kann. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100 % betragen.
Als Comonomere B) weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat; Diethylamino-methylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.
Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B) kann, bezogen auf die Gesamtmasse der. Copolymere, kann 0 bis 99,7 Gew.-% betragen und beträgt bevorzugt 0,5 bis 80 Gew.-%, besonders bevorzugt 2 bis 50 Gew.-%, betragen.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden, dass unter den kationischen Comonomeren C) auch solche zu verstehen sind, die die kationische Ladung in Form einer betainischen Struktur tragen. Comonomere C) im Sinne der Erfindung sind auch aminofunktionalisierte Precursor, die durch polymeranaloge Reaktionen (z.B. Reaktion mit DMS) in ihre entsprechenden quaternären Derivate überführt werden können.
Comonomere C) sind
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid und/oder
N-Methyl-4-vinylpyridiniumchlorid.
Der Gewichtsanteil der Comonomeren C) beträgt, bezogen auf die Gesamtmasse der Copolymere, bevorzugt 0,1 bis 99,8 Gew.-%, besonders bevorzugt 0,5 bis 30 Gew.-% und insbesondere bevorzugt 1 bis 20 Gew.-%.

Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block-(auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte siliziumhaltige Komponenten sind solche gemäß Formel (I).

R¹ -. Z- [(Si(R³R⁴)-O-)_{w}-(Si(R⁵R⁶)-O)ₓ-]-R² (I)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.
Zur Anbindung der silikonhaltigen Polymerkette an die reaktive Endgruppe R¹ ist eine geeignete chemische Brücke Z erforderlich. Bevorzugte Brücken Z sind -O-, -((C₁-C₅₀)Alkylen)-, -((C₆-C₃₀)Arylen)-, -((C₅-C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückegruppierungen Z sind -((C₁-C₁₀)Alkenyl)-(Si(OCH₃)₂)- und -(Si(OCH₃)₂)-.
Der polymere Mittelteil wird durch silikonhaltige Wiederholungseinheiten repräsentiert.
Die Reste R³, R⁴, R⁵ und R⁶ bedeuten unabhängig voneinander -CH₃; -O-CH₃, -C₆H₅ oder -O-C₆H₅.
Die Indizes w und x repräsentieren stöchiometrische Koeffizienten, die unabhängig voneinander 0 bis 500, bevorzugt 10 bis 250, betragen.
Die Verteilung der Wiederholungseinheiten über die Kette hinweg kann nicht nur rein statistisch, sondern auch blockartig, alternierend oder gradientenartig sein.
R² kann einerseits einen aliphatischen, olefinischen, cycloaliphatischen, arylaliphatischen oder aromatischen (C₁ - C₅₀)-Kohlenwasserstoffrest symbolisieren (linear oder verzweigt) oder -OH, -NH₂, -N(CH₃)₂, -R⁷ oder für die Struktureinheit [-Z-R¹] stehen: Die Bedeutung der beiden Variablen Z und R¹ wurde bereits erklärt. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃ und -O-Si(O-Si(Ph)₃)₂Ph.
Wenn R² ein Element der Gruppe [-Z-R¹] darstellt, handelt es sich um difunktionelle, Monomere, die zur Vernetzung der entstehenden Polymerstrukturen herangezogen werden können.
Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Komponenten sind die folgenden acrylische oder methacrylisch modifizierten silikonhaltigen Komponenten:

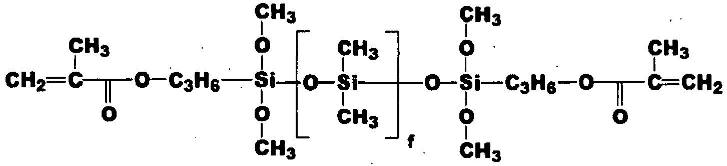

Methacryloxyproplydimethylsilyl endgeblockte Polydimethylsiloxane (f = 2 bis 500)

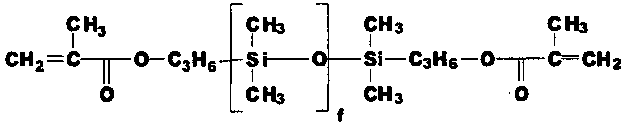

Methacryloxypropyl endgeblockte Polydimethylsiloxane (f = 2 bis 500 bis) 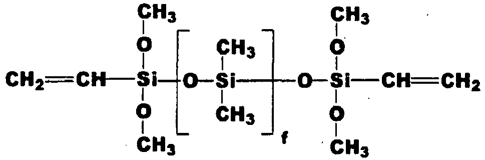 Vinyldimethoxysilyl endgeblockte Polydimethylsiloxane (f = 2-500)

Der Gewichtsanteil der Comonomeren D) kann, bezogen auf die Gesamtmasse der Copolymere, 0,1 bis 99,8 Gew.-%, bevorzugt 0,1 bis 50 Gew.-%, besonders bevorzugt 0,2 bis 40 Gew.-%, insbesondere bevorzugt 0.5 bis 30 Gew.-%, betragen.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muß die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, daß monofunktionelle Vertreter zur Bildung kammförmiger Strukturen führen, wohingegen di-, tri-, oder polyfunktionelle Komponenten E) zu zumindest teilvernetzten Strukturen führen.

Bevorzugte fluorhaltige Komponenten E) sind solche gemäß Formel (II).

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest, besonders bevorzugt einen Acryl- und Methacrylrest, dar.
Zur Anbindung der fluorhaltigen Gruppierung an die reaktive Endgruppe R¹ ist eine geeignete chemische Brücke Y erforderlich. Bevorzugte Brücken Y sind -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann.
Bei r und s handelt es sich um stöchiometrische Koeffizienten, die unabhängig voneinander Zahlen von 0 bis 200 bedeuten.

Besonders bevorzugt als fluorhaltige Komponenten sind
Perfluorhexylethanol-methacrylat,
Perfluorhexoylpropanol-methacrylat,
Perfluoroctyethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctyethanolyl-poly-[ethylglykol-blockco-propylenglycolether]-methacrylat
und/oder Perfluoroctylpropanolyl-polypropylen-glycolether-methacrylat.

Der Gewichtsanteil der Comonomeren E) kann, bezogen auf die Gesamtmasse der Copolymere, 0,1 bis 99,8 Gew.-%, bevorzugt 0,1 bis 50 Gew.-%, besonders bevorzugt 0,2 bis 30 Gew.-%, insbesondere bevorzugt 0.5 bis 20 Gew.-%, betragen.

Bei den Makromonomeren F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Copolymerisation können auch Mischungen chemisch unterschiedlicher Makromonomere F) eingesetzt werden. Bei den Makromonomeren handelt es sich um polymere Strukturen, die aus einer oder mehreren Wiederholurigseinheit(en) aufgebaut sind und eine für Polymere charakteristische Molekulargewichtsverteilung aufweisen.

Bevorzugt als Makromonomere F) sind Verbindungen gemäß Formel (III).

R¹ - Y - [(A)v - (B)_{w} - (C)ₓ - (D)_{z]} - R² (III)

R¹ stellt eine polymerisationsfähige Funktion aus der Gruppe der vinylisch ungesättigten Verbindungen darstellt, die zum Aufbau polymerer Strukturen auf radikalischem Wege geeignet sind. Bevorzugt stellt R¹ einen Vinyl-, Allyl-, Methallyl-, Methylvinyl-, Acryl-(CH₂=CH-CO-), Methacryl- (CH₂=C[CH₃]-CO-), Crotonyl-, Senecionyl-, Itaconyl-, Maleinyl-, Fumaryl- oder Styrylrest dar.
Zur Anbindung der Polymerkette an die reaktive Endgruppe ist eine geeignete verbrückende Gruppe Y erforderlich. Bevorzugte Brücken Y sind -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)-, besonders bevorzugt -0-.
Der polymere Mittelteil des Makromonomeren wird durch die diskreten Wiederholungseinheiten A, B, C und D repräsentiert. Bevorzugte Wiederholungseinheiten A,B,C und D leiten sich ab von Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid.

Die Indizes v, w, x und z in Formel (III) repräsentieren die stöchiometrische Koeffizienten betreffend die Wiederholungseinheiten A, B, C und D. v, w, x und z betragen unabhängig voneinander 0 bis 500, bevorzugt 1 bis 30, wobei die Summe der vier Koeffizienten im Mittel ≥ 1 sein muss.
Die Verteilung der Wiederholungseinheiten über die Makromonomerkette kann statistisch, blockartig, alternierend oder gradientenartig sein.
R² bedeutet einen linearen oder verzweigten aliphatischen, olefinischen, cycloaliphatischen, arylaliphatischen oder aromatischen (C₁-C₅₀)-Kohlenwasserstoffrest, OH, -NH₂, -N(CH₃)₂ oder ist gleich der Struktureinheit [-Y-R¹].
Im Falle von R² gleich [-Y-R¹] handelt es sich um difunktionelle Makromonomere, die zur Vernetzung der Copolymere geeignet sind.
Besonders bevorzugt als Makromonomere F) sind acrylisch- oder methacrylisch monofunktionalisierte Alkylethoxylate gemäß Formel (IV). 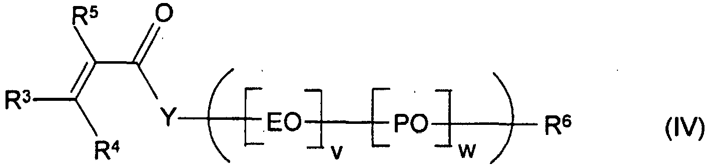 R₃, R₄, R₅ und R₆ bedeuten unabhängig voneinander Wasserstoff oder n-aliphatische, iso-aliphatische, olefinische, cycloaliphatische, arylaliphatische oder aromatische (C₁-C₃₀)-Kohlenwasserstoffreste.
Bevorzugt sind R₃ und R₄ gleich H oder -CH₃, besonders bevorzugt H; R₅ ist gleich H oder -CH₃; und R₆ ist gleich einem n-aliphatischen, iso-aliphatischen, olefinischen, cycloaliphatischen, arylaliphatischen oder aromatischen (C₁-C₃₀)-Kohlenwasserstoffrest.
v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥ 1 sein muss. Die Verteilung der EO- und PO-Einheiten über die Makromonomerkette kann statistisch, blockartig, alternierend oder gradientenartig sein. Y steht für die obengenannten Brücken, bevorzugt -O-.

Insbesondere bevorzugte Makromonomeren F) haben die folgende Struktur gemäß Formel (IV):

| Bezeichnung | R³ | R⁴ | R⁵ | R⁶ | v | w |
|---|---|---|---|---|---|---|
| ^{®}LA-030-methyacrylat | H | H | -CH₃ | -Lauryl | 3 | 0 |
| ^{®}LA-070-methacrylat | H | H | -CH₃ | -Lauryl | 7 | 0 |
| ^{®}LA-200-methacrylat | H | H | -CH₃ | -Lauryl | 20 | 0 |
| ^{®}LA-250-methacrylat | H | H | -CH₃ | -Lauryl | 25 | 0 |
| ^{®}T-080-methyacrylat | H | H | -CH₃ | -Talk | 8 | 0 |
| ^{®}T-080-acrylat | H | H | H | -Talk | 8 | 0 |
| ^{®}T-250-methyacrylat | H | H | -CH₃ | -Talk | 25 | 0 |
| ^{®}T-250-crotonat | -CH₃ | H | -CH₃ | -Talk | 25 | 0 |
| ^{®}OC-030-methacrylat | H | H | -CH₃ | -Octyl | 3 | 0 |
| ^{®}OC-105-methacrylat | H | H | -CH₃ | -Octyl | 10 | 5 |
| ^{®}Behenyl-010-methyaryl | H | H | H | -Behenyl | 10 | 0 |
| ^{®}Behenyl-020-methyaryl | H | H | H | -Behenyl | 20 | 0 |
| ^{®}Behenyl-010-senecionyl | -CH₃ | -CH₃ | H | -Behenyl | 10 | 0 |
| ^{®}PEG-440-diacrylat | H | H | H | -Acryl | 10 | 0 |
| ^{®}B-11-50-methacrylat | H | H | -CH₃ | -Butyl | 17 | 13 |
| ^{®}MPEG-750-methacrylat | H | H | -CH₃ | -Methyl | 18 | 0 |
| ^{®}P-010-acrylat | H | H | H | -Phenyl | 10 | 0 |
| ^{®}O-050-acrylat | H | H | H | -Oleyl | 5 | 0 |

Bevorzugt beträgt das Molekulargewicht der Makromonomeren F) 200 g/mol bis 10⁶ g/mol, besonders bevorzugt 150 bis 10⁴ g/mol und insbesondere bevorzugt 200 bis 5000 g/mol.

Der Gewichtsanteil des Makromonomeren F) kann, bezogen auf die Gesamtmasse der Copolymere, 0,1 bis 99,8 Gew.-%, besonders bevorzugt 2 bis 90 Gew.-%, insbesondere bevorzugt 5 bis 80 Gew.-%, betragen.

Bevorzugt als Copolymere sind solche, die durch Copolymerisation mindestens der Komponenten A), C) und D) erhältlich sind.

Weiterhin bevorzugt als Copolymere sind solche, die durch Copolymerisation mindestens der Komponenten A), C) und E) erhältlich sind.

Weiterhin bevorzugt als Copolymere sind solche, die durch Copolymerisation mindestens der Komponenten A), C) und F) erhältlich sind.

Weiterhin bevorzugt als Copolymere sind solche, die durch Copolymerisation mindestens der Komponenten A), D) und F) erhältlich sind.

Weiterhin bevorzugt als Copolymere sind solche, die durch Copolymerisation mindestens der Komponenten A), E) und F) erhältlich sind.

Weiterhin bevorzugt als Copolymere sind solche, die durch Copolymerisation mindestens der Komponenten A), B), D) und F) erhältlich sind.

Weiterhin bevorzugt als Copolymere sind solche, die durch Copolymerisation mindestens der Komponenten A), B), C), D) und F) erhältlich sind.

Die Copolymerisation wird in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.
Die Additive E) bzw. deren Mischungen müssen lediglich ganz oder teilweise im gewählten Polymerisationsmedium löslich sein. Während des eigentlichen Polymerisationsschrittes hat das Additiv G) mehrere Funktionen. Einerseits verhindert es im eigentlichen Polymerisationsschritt die Bildung übervernetzter Polymeranteile im sich bildenden Copolymerisat und andererseits wird das Additiv G) gemäß dem allgemein bekannten Mechanismus der Pfropfcopolymerisation statistisch von aktiven Radikalen angegriffen. Dies führt dazu, dass je nach Additiv G) mehr oder weniger große Anteile davon in die Copolymere eingebaut werden.

Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.
Bevorzugt als Additive G) sind in Wasser und/oder Alkoholen lösliche Homo- und Copolymere. Unter Copolymeren sind dabei auch solche mit mehr als zwei verschiedenen Monomertypen zu verstehen.
Besonders bevorzugt als Additive G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid; Polyalkylenglykole und/oder Alkylpolyglykole.
Insbesondere bevorzugt als Additive G) sind Polyvinylpyrrolidone (z.B. K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.
Das Molekulargewicht der Additive E) beträgt bevorzugt 10² bis 10⁷ g/mol, besonders bevorzugt 0,5*10⁴ bis 10⁶ g/mol.
Die Einsatzmenge des polymeren Additivs E) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.
Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.

Insbesondere bevorzugt als Vernetzer ist Trimethylolpropantriacrylat (TMPTA). Der Gewichtsanteil an vernetzenden Comonomeren, bezogen auf die Gesamtmasse der Copolymere, beträgt bevorzugt bis 20 Gew.-%, besonders bevorzugt 0,05 bis
10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodüsobutyronitril (AIBN), verwendet werden.

Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Die Polymerisationsreaktion kann z.B. als Fällungspolymerisation, Emulsionspolymerisation, Substanzpolymerisation, Lösungspölymerisation oder Gelpolymerisation geführt werden. Besonders vorteilhaft für das Eigenschaftsprofil der erfindungsgemäßen Copolymere ist die Fällungspolymerisation, bevorzugt in tert.-Butanol.
Die erfindungsgemäßen multifunktionalen Polymere besitzen eine große Strukturvielfalt und folglich breite potentielle Einsatzmöglichkeiten, die auf nahezu jede Fragestellung, bei der Grenzflächen- bzw. Oberflächeneffekte eine Rolle spielen, zugeschnitten werden können. Insbesondere soll auch auf das hohe Anwendungspotential im Bereich der Kosmetik, z.B. als Verdicker und Emulgatoren, verwiesen werden. Der Begriff "maßgeschneiderte Polymere" beschreibt bildlich die Möglichkeiten, die diese neue Polymerklasse dem Anwender an die Hand gibt.

Die nachfolgenden Beispiele dienen der Erläuterung der Erfindung, ohne sie jedoch darauf einzuschränken.

### Beispiel 1

| Reaktanden | Menge (g) |
|---|---|
| NH₃-neutralisiertes AMPS | 80 |
| Genapol-LA-070-methacrylat | 10 |
| Monofunktionalisiertes ethoxyliertes Siloxan (methacrylisch, ^{®}Silvet 7608 WITCO) | 10 |
| TMPTA | 1,8 |
| t-Butanol | 500 |
| Dilauroylperoxid (Initiator) | 1 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von DLP initiiert. Das Polymer wurde durch Absaugen des Lösungsmittels und durch anschließende Vakuumtrocknung isoliert.

### Beispiel 2

| Reaktanden | Menge (g) |
|---|---|
| NH₃-neutralisiertes AMPS | 70 |
| N-Vinylpyrrolidon | 5 |
| ^{®}Genapol-T-250-methacrylat | 15 |
| [2-(Methacryloyloxy)ethyl]trimethyl-ammoniumchlorid | 10 |
| Wasser | 500 |
| Na₂S₂O₈ (Initiator) | 1 |
| Poly-N-Vinylpyrrolidon (^{®}K-30, BASF) | 10 |

Das Polymer wurde nach dem Gelpolymerisationsverfahren in Wasser hergestellt. Dabei wurden die Monomere in Wasser gelöst, die Reaktionsmischung inertisiert und anschließend die Reaktion nach Anheizen durch Zugabe von Natriumperoxodisulfat initiiert. Das Polymergel wurde anschließend zerkleinert und das Polymer durch Vakuumtrocknung isoliert.

### Beispiel 3

| Reaktanden | Menge (g) |
|---|---|
| AMPS | 60 |
| ^{®}Genapol-BE-020-methacrylat | 10 |
| ^{®}Genapol-T-250-acrylat | 10 |
| [2-Methacryl-amido)ethyl]-trimethylammonium-chlorid | 20 |
| Cyclohexan | 200 |
| Wasser | 300 |
| Span 80 | 1 |
| Na₂S₂O₈ (Initiator) | 1 |

Das Polymer wurde nach dem Emulsionsverfahren in Wasser hergestellt. Dabei wurden die Monomere in einer Wasser/Cyclohexan unter Verwendung von ^{®}Span 80 emulgiert, die Reaktionsmischung mittels N₂ inertisiert und anschließend die Reaktion nach Anheizen durch Zugabe von Natriumperoxodisulfat gestartet. Die Polymeremulsion wurde anschließend eingedampft (Cyclohexan fungiert als Schlepper für Wasser) und dadurch das Polymer isoliert.

### Beispiel 4

| Reaktanden | Menge (g) |
|---|---|
| NH₃-neutralisiertes AMPS | 60 |
| ^{®}PEG-750-methacrylat | 20 |
| Methacryloxypyldimethicon (^{®}GP-478, Genesee Pol. Corp.) | 10 |
| Perfluoroctylpoly-ethylenglykolmethacrylat | 10 |
| t-Butanol | 500 |
| AIBN (Initiator) | 1 |
| Poly[N-vinylcaprolacton-co-acrylsäure] (10/90) | 10 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von AIBN initiiert. Das Polymer wurde durch Absaugen des Lösungsmittels und durch anschließende Vakuumtrocknung isoliert.

### Beispiel 5

| Reaktanden | Menge (g) |
|---|---|
| Na-neutralisiertes AMPS | 80 |
| N-Vinylformamid | 5 |
| ^{®}eGenapol-O-150-methacrylat | 5 |
| Diallyldimethylammoniumchlorid (DADMAC) | 10 |
| Wasser | 300 |
| TMPTA | 1,8 |
| H₂O₂/Eisen (Initiator) | 1 |
| Poly[N-Vinylformamid] | 8 |

Das Polymer wurde nach dem Lösungsverfahren in Wasser hergestellt. Dabei wurden die Monomere in Wasser gelöst, die Reaktionsmischung inertisiert und anschließend die Reaktion nach Anheizen durch ein geeignetes Redoxpaar initiiert. Die Polymerlösung wurde anschließend eingedampft und das Polymer anschließend mittels Vakuumtrocknung isoliert.

### Beispiel 6

| Reaktanden | Menge (g) |
|---|---|
| NH₃-neutralisiertes AMPS | 70 |
| ^{®}Genapol-T-250-acrylat | 10 |
| N-Methyl-4-vinylpyridiniumchlorid | 5 |
| Monofunktionalisiertes (methacrylisch) ethyoxyliertes Siloxan (^{®}Silvet Y-12867, WITCO) | 2,5 |
| Perfluorhexylpolyethylenglykolmethacrylat | 2,5 |
| Polyethylenglykoldimethacrylat (Mₙ=5000g/mol) | 10 |
| t-Butanol | 500 |
| Dilaurolyperoxid | 2 |
| Poly[N-Vinylcaprolactam] | 4 |

Das Polymer wurde nach dem Fällungsverfahren in tert. Butanol hergestellt. Dabei wurden die Monomere in t-Butanol vorgelegt, die Reaktionsmischung inertisiert und anschließend die Reaktion nach Anheizen durch Zugabe von DLP initiiert. Das Polymer wurde durch Absaugen des Lösungsmittels und durch anschließende Vakuumtrocknung isoliert.

### Beispiel 7

Dieses Polymer wurde nach dem Lösungsverfahren in tert. Butanol hergestellt. Dabei wurden die Monomere in t-Butanol vorgelegt, die Reaktionsmischung inertisiert und anschließend die Reaktion nach Anheizen durch Zugabe von DLP initiiert. Das Polymer wurde durch Abdampfen des Lösungsmittels und durch anschließende Vakuumtrocknung isoliert.

| Reaktanden | Menge (g) |
|---|---|
| NH₃-neutralisiertes AMPS | 10 |
| Acrylamid | 20 |
| N-2-Vinylpyrrolidon | 30 |
| Monofunktionalisiertes (methacrylisch) ethyoxyliertes Siloxan (^{®}Silvet 7608 WITCO) | 20 |
| Methacryloxypyldimethicon (^{®}GP-446, Genesee Pol. Corp.) | 10 |
| ^{®}Fluowet AC 812 | 10 |
| t-Butanol | 500 |
| Dilaurolyperoxid | 2 |

Das Polymer wurde nach dem Lösungsverfahren in tert. Butanol hergestellt. Dabei wurden die Monomere in t-Butanol vorgelegt, die Reaktionsmischung inertisiert und anschließend die Reaktion nach Anheizen durch Zugabe von DLP initiiert. Das Polymer wurde durch Abdampfen des Lösungsmittels und durch anschließende Vakuumtrocknung isoliert.

### Beispiel 8

| Reaktanden | Menge (g) |
|---|---|
| NH₃-neutralisiertes AMPS | 60 |
| Diallyldimethylammoniumchlorid (DADMAC) | 10 |
| [2-(Methacryloyloxy)ethyl]trimethyl-ammoniumchlorid | 10 |
| ^{®}Genapol-LA-250-crotonat | 10 |
| Methacryloxypyldimethicon (^{®}GP-478, Genesee Pol. Corp.) | 10 |
| Isopropanol | 300 |
| Wasser | 200 |
| Kaliumperoxodisulfat | 2 |
| Poly[acrylsäure-co-N-vinylformamid] | 7 |

Das Polymer wurde nach dem Lösungsverfahren in einem Isopropanol/Wasser-Gemisch hergestellt. Dabei wurden die Monomere in Isopropanol/Wasser vorgelegt, die Reaktionsmischung inertisiert und anschließend die Reaktion nach Anheizen durch Zugabe von Kaliumperoxodisulfat initiiert. Das Polymer wurde durch Abdampfen des Lösungsmittelgemisches und durch anschließende Vakuumtrocknung isoliert.

## Patentansprüche

1. Wasserlösliche oder wasserquellbare Copolymere, erhältlich durch radikalische Copolymerisation von
A) Acryloyldimethyltaurinsäure und/oder Acryloyldimethyltauraten, wobei der Neutralisationsgrad oberhalb von 80 % ist,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen, und bei denen es sich um [2-(Methacryloyloxy)ethyl]trimethylammoniumchlorid, [2-(Acryloyloxy)ethyl]trimethylammoniumchlorid, [2-Methacrylamido-ethyl]trimethylammoniumchlorid, [2-(Acrylamido)ethyl]trimethyl-ammoniumchlorid, N-Methyl-2-vinylpyridiniumchlorid und/oder N-Methyl-4-vinylpyridiniumchlorid handelt,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation in Gegenwart mindestens eines polymeren Additivs mit zahlenmittleren Molekulargewichten von 200 g/mol bis 10⁹ g/mol, bei denen es sich um Homo- oder Copolymere aus N-Vinylformamid, N-Vinylacetamid, N-Vinylpyrrolidon, Ethylenoxid, Propylenoxid, Acryloyldimethyltaurinsäure, N-Vinylcaprolacton, N-Vinylmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimethylammoniumchlorid; Polyalkylenglykole und/oder Alkylpolyglykole handelt, erfolgt,
H) mit der Maßgabe, dass die Komponente A) mit mindestens zwei Komponenten ausgewählt aus mindestens zwei der Gruppen C) bis F) copolymerisiert wird,
**dadurch gekennzeichnet, dass** der Gehalt an Acryloyldimethyltaurinsäure bzw. Acryloyldimethyltauraten, bezogen auf die Gesamtmasse der Copolymere, 50 bis 99,5 Gew.-% beträgt.

2. Copolymere nach Anspruch 1, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

3. Copolymere nach Anspruch 1 oder 2, **dadurch gekennzeichnet, dass** es sich bei den siliziumhaltigen Komponenten D) um Verbindungen der Formel (I)
R¹ - Z- [(Si(R³R⁴)-O-)_{w}-(Si(R⁵R⁶)-O)ₓ-]-R² (I)
handelt, wobei
R¹ einen Vinyl-, Allyl-, Methallyl-, Methylvinyl-, Acryl-, Methacryl-, Crotonyl-, Senecionyl-, Itaconyl-, Maleinyl-, Fumaryl- oder ein Styrylrest darstellt;
Z eine chemische Brücke, bevorzugt ausgewählt aus -O-, -((C₁-C₅₀) Alkylen)-, -((C₆-C₃₀) Arylen)-, -((C₅-C₈) Cycloalkylen)-, -((C₁-C₅₀) Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann, -((C₁-C₁₀) Alkyl)-(Si(OCH₃)₂)- und -(Si(OCH₃)₂)-, darstellt;
R³, R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen gesättigten oder ungesättigten, aliphatischen cycloaliphatischen, arylaliphatischen oder aromatischen Rest mit jeweils 1 bis 50 C-Atomen oder eine Gruppe der Formeln -OH, -NH₂, -N(CH₃)₂, -R⁷ oder eine Gruppe -Z-R¹ bedeutet, wobei Z und R¹ die obengenannten Bedeutungen haben und
R⁷ eine Gruppe der Formel -O-Si(CH₃)₃, -O-Si(Phenyl)₃, -O-Si(O-Si(CH₃)₃)₂CH₃ und -O-Si(O-Si(Ph)₃)₂Ph bedeutet.

4. Copolymere nach mindestens einem der Ansprüche 1 bis 3, **dadurch gekennzeichnet, dass** es sich bei den fluorhaltigen Komponenten E) um Verbindungen der Formel (II)
R¹-Y-CᵣH₂ᵣCₛF₂ₛCF₃ (II)
handelt, wobei
R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine chemische Brücke, bevorzugt -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃- -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten, darstellt und
r,s stöchiometrische Koeffizientendarstellen, die unabhängig voneinander Zahlen zwischen 0 und 200 sein können.

5. Copolymere nach mindestens einem der Ansprüche 1 bis 4, **dadurch gekennzeichnet, dass** es sich bei den Makromonomeren F) um Verbindungen der Formel (III) handelt,
R¹ - Y - [(A)ᵥ - (B)_{w} - (C)ₓ - (D)_{z}] - R² (III)
wobei R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine verbrückende Gruppe, bevorzugt -O-, -S-, -C(O)-, -C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃- -NH- und -N(CH₃)- darstellt;
A, B, C und D unabhängig voneinander diskrete chemische Wiederholungseinheiten, bevorzugt hervorgegangen aus Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid, insbesondere bevorzugt Ethylenoxid, Propylenoxid darstellen;
v, w, x und z unabhängig voneinander 0 bis 500, bevorzugt 1 bis 30, betragen, wobei die Summe aus v, w, x und z im Mittel ≥ 1 ist; und
R² einen linearen oder verzweigten aliphatischen, olefinischen, cycloaliphatischen, arylaliphatischen oder aromatischen (C₁-C₅₀)-Kohlenwasserstoffrest, OH, -NH₂ oder -N(CH₃)₂ darstellt oder gleich [-Y-R¹] ist.

6. Copolymere nach mindestens einem der Ansprüche 1 bis 5, **dadurch gekennzeichnet, dass** die Copolymerisation in Gegenwart mindestens eines polymeren Additivs G) erfolgt.

7. Copolymere nach mindestens einem der Ansprüche 1 bis 6, **dadurch gekennzeichnet, dass** sie vernetzt sind.

8. Copolymere nach mindestens einem der Ansprüche 1 bis 7, **dadurch gekennzeichnet, dass** sie durch Fällungspolymerisation in tert.-Butanol hergestellt werden.

## Claims

1. A water-soluble or water-swellable copolymer obtainable by free-radical copolymerization of
A) acryloyldimethyltaurine and/or acryloyldimethyltaurates, the degree of neutralization being more than 80%,
B) if desired, one or more other olefinically unsaturated, noncationic, optionally crosslinking comonomers containing at least one oxygen, nitrogen, sulfur or phosphorus atom and possessing a molecular weight of less than 500 g/mol,
C) one or more olefinically unsaturated, cationic comonomers containing at least one oxygen, nitrogen, sulfur or phosphorus atom and possessing a molecular weight of less than 500 g/mol, and which comprise [2-(methacryloyloxy)ethyl]trimethylammonium chloride,[2-(acryloyloxy)ethyl]trimethylammonium chloride, [2-methacrylamidoethyl]trimethylammonium chloride, [2-(acrylamido)ethyl]trimethylammonium chloride, N-methyl-2-vinylpyridinium chloride and/or N-methyl-4-vinylpyridinium chloride,
D) if desired, one or more at least monofunctional, silicon-containing components capable of free-radical polymerization,
E) if desired, one or more at least monofunctional, fluorine-containing components capable of free-radical polymerization,
F) if desired, one or more mono- or polyolefinically unsaturated, optionally crosslinking macromonomers each possessing at least one oxygen, nitrogen, sulfur or phosphorus atom and having a number-average molecular weight of greater than or equal to 200 g/mol, the macromonomers not being a silicon-containing component D) or fluorine-containing component E),
G) the copolymerization taking place in the presence of at least one polymeric additive having number-average molecular weights of from 200 g/mol to 10⁹ g/mol, which comprises homopolymers and copolymers of N-vinylformamide, N-vinylacetamide, N-vinylpyrrolidone, ethylene oxide, propylene oxide, acryloyldimethyltaurine, N-vinylcaprolactone, N-viriylmethylacetamide, acrylamide, acrylic acid, methacrylic acid, N-vinylmorpholide, hydroxymethyl methacrylate, diallyldimethylammonium chloride (DADMAC) and/or [2-(methacryloyloxy)ethyl]trimethylammonium chloride; polyalkylene glycols and/or alkylpolyglycols,
H) with the proviso that component A) is copolymerized with at least two components selected from at least two of the groups C) to F),
wherein the amount of acryloyldimethyltaurine and/or acryloyldimethyltaurates, based on the total mass of the copolymers, is from 50 to 99.5% by weight.

2. The copolymer as claimed in claim 1, wherein the comonomers B) comprise unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate; styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

3. The copolymer as claimed in claim 1 or 2, wherein the silicon-containing components D) are compounds of the formula (I)
R¹ - Z- [(Si(R³R⁴)-O-)_{w}-(Si(R⁵R⁶)-O)ₓ-]- R² (I)
where
R¹ represents a vinyl, allyl, methallyl, methylvinyl, acryloyl, methacryloyl, crotonyl, senecionyl, itaconyl, maleyl, fumaryl or styryl radical;
Z is a chemical bridge, preferably selected from -O-, -((C₁-C₅₀)alkylene)-, - ((C₆-C₃₀)arylene) -, - ((C₅-C₈)cycloalkylene) -, - ((C₁-C₅₀)alkenylene)-, -(polypropylene oxide)ₙ-, -(polyethylene oxide)ₒ-, -(polypropylene oxide)ₙ-(polyethylene oxide)ₒ-, where n and o independently of one another denote numbers from 0 to 200 and the distribution of the EO/PO units can be random or in the form of blocks, - ((C₁-C₁₀)alkyl)-(Si(OCH₃)₂)- and - (Si(OCH₃)₂)-;
R³, R⁴, R⁵, and R⁶ independently of one another are -CH₃, -O-CH₃, -C₆H₅ or -O-C₆H₅;
w, x denote numbers from 0 to 500, it being necessary for either w or x to be greater than zero; and
R² is a saturated or unsaturated aliphatic, cycloaliphatic, arylaliphatic or aromatic radical having in each case 1 to 50 carbon atoms or a group of the formulae -OH, -NH₂, -N(CH₃)₂ , -R⁷ or a group -Z-R¹, where Z and R¹ are as defined above and
R⁷ is a group of the formula -O-Si(CH₃)₃, -O-Si(phenyl)₃, -O-Si(O-Si(CH₃)₃)₂CH₃ or -O-Si(O-Si(Ph)₃)₂Ph.

4. The copolymer as claimed in at least one of claims 1 to 3, wherein the fluorine-containing components E) are compounds of the formula (II)
R¹-Y-CᵣH₂ᵣCₛF₂ₛCF₃ (II)
where
R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a chemical bridge, preferably -O-, -C(O)-, -C(O)-O-, -S-, -O-CH₂-CH(O-)-CH₂OH, -O-CH₂-CH(OH)-CH₂-O-, -O-SO₂-O-,
-O-S(O)-O-, -PH-, -P(CH₃)-, -PO₃-, -NH-, -N(CH₃)-, -O- (C₁-C₅₀) alkyl-O-,
-O-phenyl-O-, -O-benzyl-O-, -O-(C₅-C₈)cycloalkyl-O-,
-O-(C₁-C₅₀)alkenyl-O-, -O-(CH(CH₃)-CH₂-O)ₙ-, -O-(-CH₂-CH₂-O)ₙ-, and
-O- ([CH-CH₂-O]ₙ-[CH₂-CH₂-O]ₘ) ₒ-, where n, m, and o independently of one another denote numbers from 0 to 200; and
r, s are stoichiometric coefficients which independently of one another can be numbers between 0 and 200.

5. The copolymer as claimed in at least one of claims 1 to 4, wherein the macromonomers F) are compounds of the formula (III)
R¹ - Y - [(A)ᵥ - (B)_{w} - (C)ₓ - (D)_{z}] - R² (III)
where R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a bridging group, preferably -O-, -S-, -C(O)-, -C(O)-O-,
-O-CH₂-CH(O-)-CH₂OH, -O-CH₂-CH(OH)-CH₂O-, -O-SO₂-O-, -0-SO₂-O-,
-O-SO-O-, -PH-, -P(CH₃)-, -PO₃-, -NH-, and -N(CH₃)-;
A, B, C, and D independently of one another are discrete chemical repeating units, preferably originating from acrylamide, methacrylamide, ethylene oxide, propylene oxide, AMPS, acrylic acid, methacrylic acid, methyl methacrylate, acrylonitrile, maleic acid, vinyl acetate, styrene, 1,3-butadiene, isoprene, isobutene, diethylacrylamide, and diisopropylacrylamide, more preferably ethylene oxide or propylene oxide;
v, w, x, and z independently of one another amount to from 0 to 500, preferably from 1 to 30, the sum of v, w, x, and z being on average ≥ 1; and
R² is a linear or branched aliphatic, olefinic, cycloaliphatic, arylaliphatic or aromatic (C₁-C₅₀) hydrocarbon radical, OH, -NH₂ or -N(CH₃)₂ or is [-Y-R¹].

6. The copolymer as claimed in at least one of claims 1 to 5, wherein the copolymerization takes place in the presence of at least one polymeric additive G).

7. The copolymer as claimed in at least one of claims 1 to 6, which is crosslinked.

8. The copolymer as claimed in at least one of claims 1 to 7, prepared by precipitation polymerization in tert-butanol.

## Revendications

1. Copolymères solubles dans l'eau ou gonflants dans l'eau, pouvant être obtenus par copolymérisation radicalaire de
A) de l'acide acryloyldiméthyltaurique et/ou des taurates d'acryloyldiméthyle, le degré de neutralisation étant supérieur à 80 %,
B) éventuellement un ou plusieurs comonomères supplémentaires oléfiniquement insaturés, non cationiques, éventuellement réticulants, qui comprennent au moins un atome d'oxygène, d'azote, de soufre ou de phosphore et qui présentent un poids moléculaire inférieur à 500 g/mol,
C) un ou plusieurs comonomères cationiques oléfiniquement insaturés qui comprennent au moins un atome d'oxygène, d'azote, de soufre ou de phosphore et qui présentent un poids moléculaire inférieur à 500 g/mol, et qui sont le chlorure de [2-(méthacryloyloxy)éthyl]triméthylammonium, le chlorure de [2-(acryloyloxy)éthyl]triméthylammonium, le chlorure de [2-méthacrylamidoéthyl]triméthylammonium, le chlorure de [2-(acrylamido)éthyl]triméthylammonium, le chlorure de N-méthyl-2-vinylpyridinium et/ou le chlorure de N-méthyl-4-vinylpyridinium,
D) éventuellement un ou plusieurs composants contenant du silicium, au moins monofonctionnels, aptes à la polymérisation radicalaire,
E) éventuellement un ou plusieurs composants contenant du fluor, au moins monofonctionnels, aptes à la polymérisation radicalaire,
F) éventuellement un ou plusieurs macromonomères mono- ou polyoléfiniquement insaturés, éventuellement réticulants, qui comprennent à chaque fois au moins un atome d'oxygène, d'azote, de soufre ou de phosphore et qui présentent un poids moléculaire moyen en nombre supérieur ou égal à 200 g/mol, les macromonomères n'étant pas un composant contenant du silicium D) ou un composant contenant du fluor E),
G) la copolymérisation ayant lieu en présence d'au moins un additif polymère de poids moléculaire moyen en nombre de 200 g/mol à 10⁹ g/mol, qui consiste en des homo- ou copolymères de N-vinylformamide, N-vinylacétamide, N-vinylpyrrolidone, oxyde d'éthylène, oxyde de propylène, acide acryloyldiméthyltaurique, N-vinylcaprolactone, N-vinylméthylacétamide, acrylamide, acide acrylique, acide méthacrylique, N-vinylmorpholide, méthacrylate d'hydroxyméthyle, chlorure de diallyldiméthylammonium (DADMAC) et/ou chlorure de [2-(méthacryloyloxy)éthyl]triméthylammonium ; des polyalkylène glycols et/ou des alkylpolyglycols,
H) à condition que le composant A) soit copolymérisé avec au moins deux composants choisis dans au moins deux des groupes C) à F),
**caractérisés en ce que** la teneur en acide acryloyldiméthyltaurique ou en taurates d'acryloyldiméthyle, par rapport à la masse totale des copolymères, est de 50 à 99,5 % en poids.

2. Copolymères selon la revendication 1, **caractérisés en ce que** les comonomères B) sont des acides carboxyliques insaturés, des sels d'acides carboxyliques insaturés, des anhydrides d'acides carboxyliques insaturés, des esters d'acides carboxyliques insaturés avec des alcools aliphatiques, oléfiniques, cycloaliphatiques, arylaliphatiques ou aromatiques de 1 à 22 atomes C, des N-vinylamides à chaîne ouverte, des N-vinylamides cycliques d'une taille de cycle de 3 à 9, des amides de l'acide acrylique, des amides de l'acide méthacrylique, des amides d'acides acryliques substitués, des amides d'acides méthacryliques substitués, la 2-vinylpyridine, la 4-vinylpyridine, l'acétate de vinyle ; le styrène, l'acrylonitrile, le chlorure de vinyle, le chlorure de vinylidène, le tétrafluoroéthylène, l'acide vinylphosphonique ou ses esters ou sels, l'acide vinylsulfonique ou ses esters ou sels, l'acide allylphosphonique ou ses esters ou sels et/ou l'acide méthallylsulfonique ou ses esters ou sels.

3. Copolymères selon la revendication 1 ou 2, **caractérisés en ce que** les composants contenant du silicium D) sont des composés de Formule (I)
R¹-Z-[(Si(R³R⁴)-O-)_{w}-(Si(R₅R⁶)-O)ₓ-]-R² (I)
dans laquelle
R¹ représente un radical vinyle, allyle, méthallyle, méthylvinyle, acryle, méthacryle, crotonyle, sénécionyle, itaconyle, maléinyle, fumaryle ou styryle ;
Z représente un pont chimique, de préférence choisi parmi -O-, - (alkylène en (C₁-C₅₀))-, -(arylène en (C₆-C₃₀))-, -(cycloalkylène en (C₅-C₈))-, -(alcénylène en (C₁-C₅₀))-, -(oxyde de polypropylène)n-, -(oxyde de polyéthylène)ₒ-, -(oxyde de polypropylène)ₙ(oxyde de polyéthylène)ₒ-, n et o signifiant indépendamment l'un de l'autre des nombres de 0 à 200 et la distribution des unités EO/PO pouvant être statistique ou séquencée, -(alkyle en (C₁-C₁₀))-(Si(OCH₃)₂)- et - (Si (OCH₃)₂)- ;
R³, R⁴, R⁵ et R⁶ signifient indépendamment les uns des autres -CH₃, -O-CH₃, -C₆H₅ ou -O-C₆H₅ ;
w, x signifient des nombres de 0 à 500, w ou x devant être supérieur à zéro, et
R² signifie un radical saturé ou insaturé, aliphatique, cycloaliphatique, arylaliphatique ou aromatique, contenant à chaque fois 1 à 50 atomes C, ou un groupe de formule -OH, -NH₂, -N(CH₃)₂, -R⁷ ou un groupe -Z-R¹, Z et R¹ ayant les significations indiquées précédemment, et
R⁷ signifiant un groupe de formule -O-Si(CH₃)₃, -0-Si(phényle)₃, -O-Si (O-Si (CH₃)₃)₂CH₃ et -O-Si(O-Si(Ph)₃)₂Ph.

4. Copolymères selon au moins l'une quelconque des revendications 1 à 3, **caractérisés en ce que** les composants contenant du fluor E) sont des composés de Formule (II)
R¹-Y-CᵣH₂ᵣCₛF₂ₛCF₃ (II)
dans laquelle
R¹ représente une fonction polymérisable du groupe des composés vinyliquement insaturés, de préférence un radical vinyle, allyle, méthallyle, méthylvinyle, acryle, méthacryle, crotonyle, sénécionyle, itaconyle, maléinyle, fumaryle ou styryle ;
Y représente un pont chimique, de préférence -O-,-C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-,-PO₃-, -NH-, -N(CH₃)-, -O-alkyle en (C₁-C₅₀)-O-, -O-phényl-O-, -O-benzyl-O-, -O-cycloalkyle en (C₅-C₈)-O-,-O-alcényle en (C₁-C₅₀)-O-, -O-(CH(CH₃)-CH₂-O)ₙ-, -O-(CH₂-CH₂-O)ₙ- et -O- ([CH-CH₂-O]ₙ-[CH₂-CH₂-O]ₘ)₀-, n, m et o signifiant indépendamment les uns des autres des nombres de 0 à 200, et
r, s représentant des coefficients stoechiométriques qui peuvent être indépendamment l'un de l'autre des nombres compris entre 0 et 200.

5. Copolymères selon au moins l'une quelconque des revendications 1 à 4, **caractérisés en ce que** les macromonomères F) sont des composés de Formule (III)
R¹-Y-[(A)ᵥ-(B)_{w}-(C)ₓ-(D)_{z}]-R² (III)
dans laquelle R¹ représente une fonction polymérisable du groupe des composés vinyliquement insaturés, de préférence un radical vinyle, allyle, méthallyle, méthylvinyle, acryle, méthacryle, crotonyle, sénécionyle, itaconyle, maléinyle, fumaryle ou styryle ;
Y représente un groupe pontant, de préférence -O-, -S-, -C(O)-, -C(O)-O-, -O-CH₂-CH(O-)-CH₂OH, -O-CH₂-CH (OH)-CH₂O-, -O-SO₂-O-, -O-SO2-O-, -O-SO-O-, -PH-, -P(CH₃)-,-PO₃-, -NH- et -N(CH₃)-;
A, B, C et D représentent indépendamment les uns des autres des unités de répétition chimiques discrètes, de préférence provenant d'acrylamide, de méthacrylamide, d'oxyde d'éthylène, d'oxyde de propylène, d'AMPS, d'acide acrylique, d'acide méthacrylique, de méthacrylate de méthyle, d'acrylonitrile, d'acide maléique, d'acétate de vinyle, de styrène, de 1,3-butadiène, d'isoprène, d'isobutène, de diéthylacrylamide et de diisopropylacrylamide, de manière particulièrement préférée d'oxyde d'éthylène, d'oxyde de propylène ;
v, w, x et z représentent indépendamment les uns des autres 0 à 500, de préférence 1 à 30, la somme de v, w, x et z étant en moyenne 1 ; et
R² représente un radical hydrocarboné en (C₁-C₅₀) linéaire ou ramifié, aliphatique, oléfinique, cycloaliphatique, arylaliphatique ou aromatique, OH,-NH₂ ou -N(CH₃)₂ ou [-Y-R¹].

6. Copolymères selon au moins l'une quelconque des revendications 1 à 5, **caractérisés en ce que** la copolymérisation a lieu en présence d'au moins un additif polymère G).

7. Copolymères selon au moins l'une quelconque des revendications 1 à 6, **caractérisés en ce qu'**ils sont réticulés.

8. Copolymères selon au moins l'une quelconque des revendications 1 à 7, **caractérisés en ce qu'**ils sont fabriqués par polymérisation par précipitation dans du tert.-butanol.
